(19) Europäisches Patentamt
European Patent Office
Office européen des brevets

(11) Veröffentlichungsnummer: **0 170 624**
**B1**

(12) # EUROPÄISCHE PATENTSCHRIFT

(45) Veröffentlichungstag der Patentschrift: **07.01.88**

(51) Int. Cl.⁴: **C 07 C 149/273,** C 09 K 15/14, C 08 K 5/37, C 10 M 135/24

(21) Anmeldenummer: **85810351.8**

(22) Anmeldetag: **29.07.85**

(54) **2,4,6-Trifunktionalisierte Phenole.**

(30) Priorität: **02.08.84 CH 3729/84**

(43) Veröffentlichungstag der Anmeldung: **05.02.86 Patentblatt 86/6**

(45) Bekanntmachung des Hinweises auf die Patenterteilung: **07.01.88 Patentblatt 88/1**

(84) Benannte Vertragsstaaten: **BE DE FR GB IT**

(56) Entgegenhaltungen:
**US - A - 2 322 376**
**US - A - 3 660 352**
**US - A - 3 903 173**
**US - A - 4 091 037**

(73) Patentinhaber: **CIBA-GEIGY AG, Klybeckstrasse 141, CH-4002 Basel (CH)**

(72) Erfinder: **Evans, Samuel, Route des Charbonnières 17, CH-1723 Marly (CH)**
Erfinder: **Meier, Hans-Rudolf, Dr., Ch. des Epinettes 12, CH-1723 Marly (CH)**

**EP 0 170 624 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2,4,6-trifunktionalisierte Phenole, Zusammensetzungen enthaltend diese Verbindungen sowie deren Verwendung zum Stabilisieren von organischem Material.

Trifunktionalisierte thiomethylhaltige Phenole sind bekannt. So werden in Azerb. Khim. Zh., 1973 (5-6), 66-69 spezielle Verbindungen dieses Typs als Antioxidantien für Polymere beschrieben.

In der US-PS 2 417 118 werden unter anderem tris-alkylthiomethylhaltige Phenole als Zusätze zu Mineralölen beschrieben.

Die vorliegende Erfindung betrifft Verbindungen der Formel I

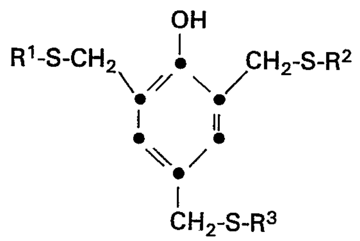

(I)

worin $R^1$, $R^2$ und $R^3$ unabhängig voneinander durch ein oder zwei Hydroxygruppen substituiertes $C_2$-$C_{20}$ Alkyl, $-C(R^4R^5)-(CHR^6)_n-W$, $-(CH_2)_2-OCOR^{10}$ oder ein Rest

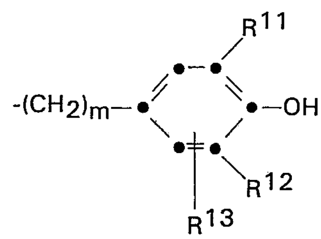

bedeuten, worin $R^4$, $R^5$ und $R^6$ unabhängig voneinander Wasserstoff, $C_1$-$C_6$ Alkyl, Phenyl oder Cyclohexyl sind, W $-COR^7$, $-COOR^8$, $-CON(R^{15}R^9)$ oder -CN ist, wobei $R^7$ Wasserstoff, $C_1$-$C_{20}$ Alkyl, $C_5$-$C_{12}$ Cycloalkyl, Phenyl, 1-Naphthyl, 2-Naphthyl, $C_7$-$C_{14}$ Aralkyl oder $C_7$-$C_{14}$ Alkaryl bedeutet, $R^8$ $C_1$-$C_{20}$ Alkyl, $C_5$-$C_{12}$ Cycloalkyl, Phenyl, 1- oder 2-Naphthyl, $C_7$-$C_{14}$ Aralkyl, $C_7$-$C_{14}$ Alkaryl oder durch eine Hydroxy- oder Cyangruppe substituiertes$C_2$-$C_{20}$ Alkyl ist, oder $C_2$-$C_{12}$ Alkenyl, $C_2$-$C_{12}$ Alkinyl, durch ein oder zwei $-NO_2$, -Cl, -Br, $-OCH_3$ oder $-COOR^{14}$ substituiertes Phenyl bedeutet, oder ein Rest

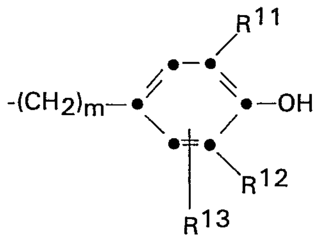

ist, oder durch ein bis fünf -O-, -S-, $-N(CH_3)-$, $-N(C_2H_5)-$ oder $-SO_2-$ unterbrochenes gegebenenfalls mit einer Hydroxygruppe substituiertes $C_3$-$C_{20}$ Alkyl bedeutet, wobei gegebenenfalls mehrfach auftretende Heteroatome durch mindestens eine Methylengruppe getrennt sein müssen, $R^9$ eine der Bedeutungen von $R^8$ besitzt oder zusätzlich Wasserstoff bedeutet, $R^{15}$ eine der Bedeutungen von $R^9$ besitzt oder worin $R^9$ und $R^{15}$ zusammen mit dem gemeinsamen Stickstoffatom einen fünf-, sechs- oder siebengliedrigen heterocyclischen Ring bilden, der gegebenenfalls noch ein weiteres Heteroatom enthalten kann, $R^{10}$ $C_1$-$C_{20}$ Alkyl, $C_5$-$C_{12}$ Cycloalkyl, Phenyl, 1- oder 2-Naphthyl, $C_7$-$C_{14}$ Aralkyl, $C_7$-$C_{14}$ Alkaryl oder einen Rest

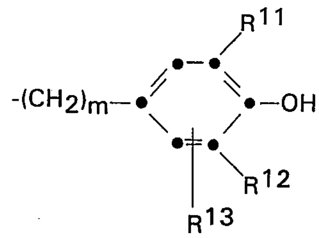

bedeutet, m 0, 1 oder 2 ist, n für 0 oder 1 steht, $R^{11}$, $R^{12}$ oder $R^{13}$ unabhängig voneinander Wasserstoff, $C_1$-$C_{20}$ Alkyl, Cyclohexyl oder Phenyl sind, und schliesslich $R^{14}$ $C_1$-$C_6$ Alkyl, Phenyl, Cyclohexyl, Benzyl oder Tolyl ist.

$R^1$, $R^2$ oder $R^3$ als durch ein oder zwei Hydroxygruppen substituiertes $C_2$-$C_{20}$ Alkyl bedeuten beispielsweise 2-Hydroxyethyl, 2-Hydroxypropyl, 2-Hydroxybutyl, 2-Hydroxyhexyl, 2-Hydroxyoctyl, 2-Hydroxydecyl, 2-Hydroxydodecyl, 2-hydroxytetradecyl, 2-Hydroxyhexadecyl, 2-Hydroxyoctadecyl, 2-Hydroxyeicosyl oder 2,3-Dihydroxypropyl. Bevorzugt werden 2-Hydroxyethyl, 2-Hydroxypropyl oder 2,3-Dihydroxypropyl.

$R^7$, $R^8$, $R^9$, $R^{10}$, $R^{11}$, $R^{12}$, $R^{13}$ oder $R^{15}$ bedeuten als $C_1$-$C_{10}$ Alkyl beispielsweise Methyl, Ethyl, n-Propyl, Isopropyl, n-Butyl, sek.-Butyl, tert.-Butyl, n-Pentyl, Isopentyl, n-Hexyl, 1,1-Dimethylbutyl, n-Heptyl, n-Octyl, 2-Ethylhexyl, n-Nonyl, n-Decyl, 1,1,3,3-Tetramethylbutyl, 1,1,3,3-Tetramethylhexyl, n-Undecyl, n-Dodecyl, 1,1,3,3,5,5-Hexamethylhexyl, 2,2,4,6,6-Pentamethylhept-4-yl, n-Tetradecyl, n-Hexadecyl, n-Octadecyl oder n-Eicosyl.

$R^7$, $R^8$, $R^9$, $R^{10}$, $R^{11}$, $R^{12}$, $R^{13}$ oder $R^{15}$ sind vorzugsweise $C_1$-$C_{12}$ Alkyl und bedeuten dann beispielsweise Methyl, Ethyl, n-Propyl, Isopropyl, n-Butyl, sek.-Butyl, tert.-Butyl, n-Pentyl, Isoamyl, n-Hexyl, n-Heptyl, n-Octyl, 1,1,3,3-Tetramethylbutyl, n-Nonyl, n-Decyl, n-Dodecyl, 2,2,4,6,6-Pentamethylhept-4-yl oder 1,1,3,3,5,5-Hexamethylhexyl.

$R^{11}$, $R^{12}$ oder $R^{13}$ sind ganz besonders bevorzugt verzweigtes $C_3$-$C_{12}$ Alkyl und sind als solches beispielsweise Isopropyl, sek.-Butyl, tert.-Butyl, tert.-Pentyl, 1,1-Dimethylbutyl, 2-Ethylhexyl, 1,1,3,3-Tetramethylbutyl, 1,1,3,3-Tetramethylhexyl, 2,2,4,6,6-Pentamethylhept-4-yl oder 1,1,3,3,5,5-Hexamethylhexyl; insbesondere jedoch tert.-Butyl.

$R^4$, $R^5$, $R^6$ und $R^{14}$ als $C_1$-$C_6$ Alkyl bedeuten beispielsweise Methyl, Ethyl, n-Propyl, Isopropyl, n-Butyl, sek.-Butyl, tert.-Butyl, n-Pentyl, Isopentyl oder n-Hexyl. Besonders bevorzugt sind geradkettige $C_1$-$C_6$ Alkylreste und ganz besonders bevorzugt Methyl.

$R^7$, $R^8$, $R^9$, $R^{10}$ oder $R^{15}$ als $C_5$-$C_{12}$ Cycloalkyl bedeuten beispielsweise Cyclopentyl, Cyclohexyl, Cycloheptyl, Cyclooctyl, Cyclononyl, Cyclodecyl, Cycloundecyl oder Cyclododecyl. Bevorzugt werden $C_5$-$C_9$ Cycloalkyl und ganz besonders Cyclohexyl.

$R^8$, $R^9$ und $R^{15}$ als $C_2$-$C_{12}$ Alkenyl bedeuten beispielsweise Vinyl, Allyl, But-3-enyl, Pent-4-enyl, Hex-5-enyl, Oct-7-enyl, Dec-9-enyl oder Dodec-11-enyl. Bevorzugt werden Vinyl oder Allyl. $R^8$ und $R^9$ als $C_2$-$C_{12}$ Alkinyl bedeuten beispielsweise Ethinyl, Propargyl, But-3-inyl, Hex-5-inyl, Oct-7-inyl, Dec-9-inyl oder Dodec-11-inyl. Bevorzugt wird Propargyl.

$R^7$, $R^8$, $R^9$, $R^{10}$ oder $R^{15}$ als $C_7$-$C_{14}$ Aralkyl bedeuten beispielsweise Benzyl, Phenylethyl, α-Methylbenzyl, α,α-Dimethylbenzyl, Phenylbutyl, Phenyl-α,α-dimethylpropyl, Phenylhexyl, Phenyl-α,α-dimethylbutyl, Phenyloctyl oder Phenyl-α,α-dimethylhexyl. Bevorzugt wird Benzyl.

$R^7$, $R^8$, $R^9$, $R^{10}$ oder $R^{15}$ als $C_7$-$C_{14}$ Alkaryl bedeuten beispielsweise o-, m- oder p-Tolyl, 2,3-, 2,4-, 2,5-, 2,6-, 3,4-, 3,5- oder 3,6-Dimethylphenyl, o-, m- oder p-Ethylphenyl, o-, m- oder p-Propylphenyl, o-, m- oder p-Isopropylphenyl, o-, m- oder p-Butylphenyl, o-, m- oder p-sek.-Butylphenyl, o-, m- oder p-tert.-Butylphenyl, 2,3-, 2,4-, 2,5-, 2,6-, 3,4-, 3,5- oder 3,6-Dibutylphenyl bzw. -Di-tert.-butylphenyl oder o-, m- oder p-Hexylphenyl oder o-, m- oder p-Octylphenyl. Bevorzugt werden o-, m- oder p-Tolyl.

$R^8$, $R^9$ oder $R^{15}$ als durch eine Hydroxy- oder Cyangruppe substituiertes $C_2$-$C_{20}$ Alkyl bedeuten beispielsweise 2-Hydroxyethyl, 2-Hydroxypropyl, 2-Hydroxybutyl, 2-Hydroxyhexyl, 2-Hydroxyoctyl, 2-Hydroxydecyl, 2-Hydroxydodecyl, 2-Hydroxytetradecyl, 2-Hydroxyhexadecyl, 2-Hydroxyoctadecyl, 2-Hydroxyeicosyl oder 2-Cyanethyl. Bevorzugt werden 2-Hydroxyethyl, 2-Hydroxypropyl oder 2-Cyanethyl.

$R^8$, $R^9$ oder $R^{15}$ als durch ein bis fünf -O-, -S-, -$N(CH_3)$-, $N(C_2H_5)$- oder -$So_2$- unterbrochenes gegebenenfalls mit einer Hydroxygruppe substituiertes $C_3$-$C_{20}$ Alkyl bedeuten beispielsweise 3-Oxabutyl, 3,6-Dioxaheptyl, 3,6,9-Trioxadecyl, 3,6,9,12-Tetraoxatridecyl, 3,6,9,12,15-Pentaoxyhexadecyl, 3,6,9,12,15,18-Hexaoxanonadecyl, 5-Hydroxy-3-oxapentyl, 8-Hydroxy-3,6-dioxaoctyl, 11-Hydroxy-3,6,9-trioxaundecyl, 14-Hydroxy-3,6,9,12-tetraoxatetradecyl, 3-Thiabutyl, 5-Hydroxy-3-azamethylpentyl, 5-Hydroxy-3-azaethylpentyl, 3-Azamethyl-6-oxaheptyl, 3-Azaethyl-6-oxaheptyl, 3-Azamethylbutyl oder 3-Azaethylbutyl. Bevorzugt werden durch -O- unterbrochene $C_3$-$C_{12}$ Alkylreste, die gegebenenfalls mit einer Hydroxygruppen substituiert sind. Ganz besonders bevorzugt werden 5-Hydroxy-3-oxapentyl oder 3-Oxabutyl.

Als durch ein oder zwei -$NO_2$, -Cl, -Br, -$OCH_3$ oder -$COOR^{14}$ substituiertes Phenyl bedeuten $R^8$, $R^9$ oder $R^{15}$ beispielsweise o-, m- oder p-Nitrophenyl, -Chlorphenyl, -Bromphenyl, -Methoxyphenyl, -Methoxycarbonylphenyl, Ethoxycarbonylphenyl, 2,3-, 2,4-, 2,5-, 2,6-, 3,4- oder 3,5-Dinitrophenyl, -Dichlorphenyl, -Dibromphenyl, -Dimethoxyphenyl, -Dimethoxycarbonylphenyl oder -Diethoxycarbonylphenyl. Bevorzugt werden o-, m- oder p-Chlorphenyl, -Methoxyphenyl oder -Methoxycarbonylphenyl.

Bilden $R^9$ und $R^{15}$ zusammen mit dem gemeinsamen Stickstoffatom einen fünf-, sechs- oder siebengliedrigen heterocyclischen Ring, der gegebenenfalls noch ein weiteres Heteroatom enthalten kann, so handelt es sich bei diesem Ring beispielsweise um Pyrrol, 2-H-Pyrrol, Imidazol, Pyrazol, Pyrrolidin, Pyrrolin, Imidazolidin, Imidazolin, Pyrazolidin, Pyrazolin, Piperidin, Piperazin, Hexamethylenimin oder Morpholin. Bevorzugt werden Pyrrol, Pyrrolidin, Hexamethylenimin, Piperidin oder Morpholin und ganz besonders Piperidin oder Morpholin.

Besonders bevorzugt werden Verbindungen der Formel I, worin $R^1$, $R^2$ und $R^3$ unabhängig voneinander 2-Hydroxyethyl, 2-Hydroxypropyl oder 2,3-Dihydroxypropyl sind.

Ebenfalls von Interesse sind Verbindungen der Formel I, worin $R^1$, $R^2$ und $R^3$ unabhängig voneinander -$C(R^4R^5)$-$(CHR^6)_n$-W, -$(CH_2)_2$-$OCOR^{10}$ oder einen Rest

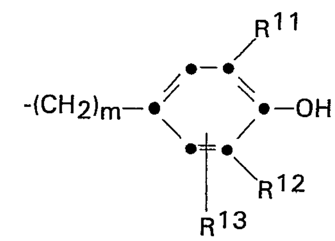

bedeuten, wobei die Reste W, $R^4$, $R^5$, $R^6$, $R^{10}$, $R^{11}$, $R^{12}$ und $R^{13}$ sowie die Indices m und n die oben angegebenen Bedeutungen besitzen.

Besonders bevorzugt werden Verbindungen der Formel I, worin $R^1$, $R^2$ und $R^3$ unabhängig voneinander -$C(R^4R^5)$-$(CHR^6)_n$-W, -$(CH_2)_2$-$OCOR^{10}$ oder einen Rest

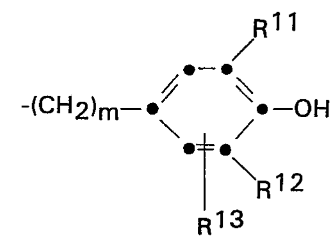

bedeuten, worin $R^4$, $R^5$ und $R^6$ unabhängig voneinander Wasserstoff oder Methyl sind, W -$COR^7$, -$COOR^8$, -$CON(R^9R^{15})$ oder -CN bedeutet, worin $R^7$ $C_{19}$-$C_{12}$ Alkyl, $C_5$-$C_9$ Cycloalkyl, Phenyl, Benzyl oder Tolyl ist, $R^8$, $R^9$ und $R^{15}$ die gleiche Bedeutung wie $R^7$ besitzen oder zusätzlich 2-Hydroxyethyl, 2-Hydroxypropyl oder 2-Cyanethyl, Allyl, Propargyl, durch ein -Cl, -$COOCH_3$ oder -$OCH_3$ substituiertes Phenyl oder ein Rest

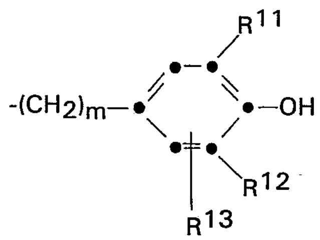

bedeuten, oder worin $R^8$, $R^9$ und $R^{15}$ durch ein bis fünf -O- unterbrochenes gegebenenfalls mit einer Hydroxygruppe substituiertes $C_3$-$C_{12}$ Alkyl darstellen, wobei gegebenenfalls mehrfach auftretende Sauerstoffatome durch mindestens eine Methylengruppe getrennt sein müssen, oder worin $R^9$ und $R^{15}$ zusammen mit dem gemeinsamen Stickstoffatom einen Pyrrol-, Pyrrolidin-, Piperidin- oder Morpholinring bilden, $R^{10}$ $C_1$-$C_{12}$ Alkyl, $C_5$-$C_9$ Cycloalkyl, Phenyl $C_7$-$C_9$ Aralkyl oder ein Rest

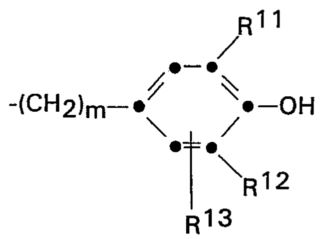

bedeutet, m 0, 1 oder 2 und n 0 oder 1 sind, und schliesslich $R^{11}$, $R^{12}$ und $R^{13}$ unabhängig voneinander Wasserstoff oder $C_1$-$C_{12}$ Alkyl sind.

Ebenfalls von Interesse sind Verbindungen der Formel I, worin $R^1$, $R^2$ und $R^3$ unabhängig voneinander $-C(R^4R^5)-(CHR^6)_n-W$, $-(CH_2)_2-OCOR^{10}$ oder ein Rest

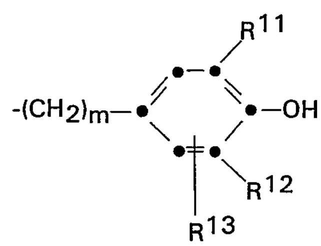

bedeuten, $R^4$, $R^5$ und $R^6$ unabhängig voneinander Wasserstoff oder Methyl sind, W $-COOR^8$ oder $-CON(R^9R^{15})$ bedeutet, wobei $R^8$, $R^9$ und $R^{15}$ unabhängig voneinander $C_1$-$C_{12}$ Alkyl, Cycloalkyl, Phenyl, Benzyl, Tolyl oder einen Rest

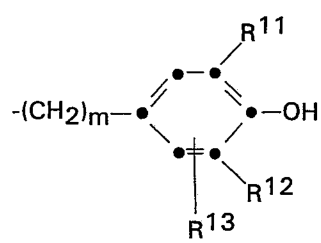

bedeuten, oder worin $R^8$, $R^9$ und $R^{15}$ durch ein bis fünf -O- unterbrochenes gegebenenfalls durch eine Hydroxygruppe substituiertes $C_3$-$C_{12}$ Alkyl sind, wobei gegebenenfalls mehrfach auftretende Sauerstoffatome durch mindestens eine Methylengruppe getrennt sein müssen, $R^{10}$ $C_1$-$C_{12}$ Alkyl, Cyclohexyl, Phenyl, Benzyl oder ein Rest

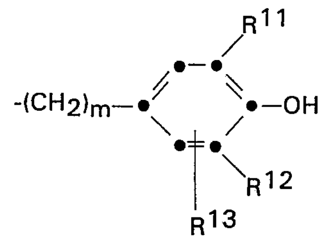

bedeutet, m 0, 1 oder 2 und n 0 oder 1 sind, und $R^{11}$, $R^{12}$ und $R^{13}$ unabhängig voneinander für Wasserstoff oder tert.-Butyl stehen.

Ganz besonders bevorzugt werden Verbindungen der Formel I, worin $R^1$, $R^2$ und $R^3$ $-C(R^4R^5)-(CHR^6)_n-W$ bedeuten, $R^4$, $R^5$ und $R^6$ unabhängig voneinander Wasserstoff oder Methyl sind, W $-COOR^8$ oder $-CON(R^9R^{15})$ ist, $R^8$, $R^9$ und $R^{15}$ unabhängig voneinander $C_1$-$C_{12}$ Alkyl, Cyclohexyl, Phenyl, Benzyl, Tolyl oder ein Rest

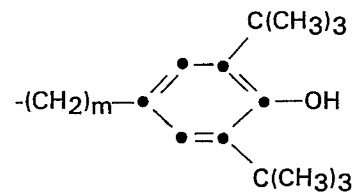

bedeuten, oder 2-Hydroxyethyl, 2-Hydroxypropyl, 3-Oxabutyl oder ein Rest $-(CH_2-CH_2-O)—CH_2-CH_2-OH$ sind, wobei p 1, 2 oder 3 bedeutet, oder worin $R^9$ und $R^{15}$ zusammen mit dem gemeinsamen Stickstoffatom einen Piperidin- oder Morpholinring bilden und worin m 0, 1 oder 2 und n 0 oder 1 sind.

Ebenfalls Beachtung finden Verbindungen der Formel I, worin $R^1$, $R^2$ und $R^3$ $-(CH_2)_2-OCOR^{10}$ bedeuten, $R^{10}$ $C_1$-$C_{12}$ Alkyl, Cycloalkyl, Phenyl, Benzyl oder ein Rest

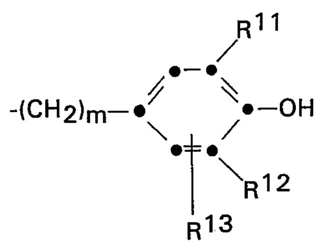

ist, worin $R^{11}$, $R^{12}$ und $R^{13}$ unabhängig voneinander Wasserstoff oder tert.-Butyl sind, und worin m 0, 1 oder 2 bedeutet.

Weiterhin sind Verbindungen der Formel I von Interesse, worin $R^1$, $R^2$ und $R^3$ ein Rest

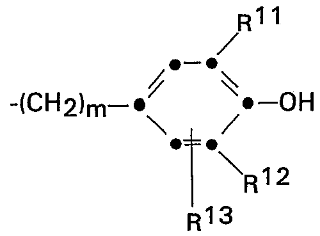

bedeuten, worin m 0, 1 oder 2 ist und $R^{11}$, $R^{12}$ und $R^{13}$ unabhängig voneinander Wasserstoff oder tert.-Butyl sind.

Als Beispiele für Verbindungen der Formel I sind die nachfolgend aufgezählten Substanzen anzusehen:

2,4,6-Tris-(2-hydroxyethylthiomethyl)-phenol
2,4,6-Tris-(2,3-dihydroxypropylthiomethyl)-phenol
Trimethylester des 2,4,6-Tris-(3-carboxy-2-thiapropyl)-phenols
Triethylester des 2,4,6-Tris-(3-carboxy-2-thiapropyl)-phenols
Tri-n-butylester des 2,4,6-Tris-(3-carboxy-2-thiapropyl)-phenols
Tri-n-hexylester des 2,4,6-Tris-(3-carboxy-2-thiapropyl)-phenols
Tri-n-octylester des 2,4,6-Tris-(3-carboxy-2-thiapropyl)-phenols
Tri-(2-ethylhexyl)-ester des 2,4,6-Tris-(3-carboxy-2-thiapropyl)-phenols
Tri-n-decylester des 2,4,6-Tris-(3-carboxy-2-thiapropyl)-phenols
Tri-n-dodecylester des 2,4,6-Tris-(3-carboxy-2-thiapropyl)-phenols
Tri-n-tetradecylester des 2,4,6-Tris-(3-carboxy-2-thiapropyl)-phenols
Tri-n-hexadecylester des 2,4,6-Tris-(3-carboxy-2-thiapropyl)-phenols
Tri-n-octadecylester des 2,4,6-Tris-(3-carboxy-2-thiapropyl)-phenols
Tri-n-eicosylester des 2,4,6-Tris-(3-carboxy-2-thiapropyl)-phenols
Trioleylester des 2,4,6-Tris-(3-carboxy-2-thiapropyl)-phenols
Tricyclohexylester des 2,4,6-Tris-(3-carboxy-2-thiapropyl)-phenols
Triphenylester des 2,4,6-Tris-(3-carboxy-2-thiapropyl)-phenols
Tribenzylester des 2,4,6-Tris-(3-carboxy-2-thiapropyl)-phenols
Tri-α-methylbenzylester des 2,4,6-Tris-(3-carboxy-2-thiapropyl)-phenols
Tri-(α,α-dimethylbenzyl)-ester des 2,4,6-Tris-(3-carboxy-2-thiapropyl)-phenols
Tri-p-tolylester des 2,4,6-Tris-(3-carboxy-2-thiapropyl)-phenols
Tri-(3-oxabutyl)-ester des 2,4,6-Tris-(3-carboxy-2-thiapropyl)-phenols
Tri-(3-thiabutyl)-ester des 2,4,6-Tris-(3-carboxy-2-thiapropyl)-phenols
Tri-(N,N-dimethylamid) des 2,4,6-Tris-(3-carboxy-2-thiapropyl)-phenols
Tri-(N,N-di-[2-ethylhexyl]-amid) des 2,4,6-Tris-(3-carboxy-2-thiapropyl)-phenols
Triamid des 2,4,6-Tris-(3-carboxy-2-thiapropyl)-phenols
2,4,6-Tri-(4-acetyl-2-thiabutyl)-phenol
2,4,6-Tri-(4-cyano-2-thiabutyl)-phenol
Trimethylester des 2,4,6-Tris-(4-carboxy-2-thiabutyl)-phenols
Tri-n-octylester des 2,4,6-Tris-(4-carboxyl-2-thiabutyl)-phenols
Tri-(2-ethylhexyl)-ester des 2,4,6-Tris-(4-carboxy-2-thiabutyl)-phenols
Tri-n-dodecylester des 2,4,6-Tris-(4-carboxy-2-thiabutyl)-phenols
Tri-(2-ethylhexyl)-ester des 2,4,6-Tris-(4-carboxy-2-thiapentyl)-phenols
2,4,6-Tri-(4-acetoxy-2-thiabutyl)-phenol
2,4,6-Tri-(4-benzoyloxy-2-thiabutyl)-phenol
2,4,6-Tri-(4-cyclohexylcarbonyloxy-2-thiabutyl)-phenol
Tri-[N-(2-hydroxyethyl)]-amid des 2,4,6-Tris-(3-carboxy-2-thiapropyl)-phenols
Tri-(2-hydroxyethyl)-ester des 2,4,6-Tris-(3-carboxy-2-thiapropyl)-phenols
Tri-(N-piperidyl)-amid des 2,4,6-Tris-(3-carboxy-2-thiapropyl)-phenols
2,4,6-Tris-(4-benzylcarbonyloxy-2-thiabutyl)-phenol
2,4,6-Tris-{4-[3-(4-hydroxy-3,5-di-tert.-butylphenyl)propionyloxy]-2-thiabutyl}-phenol
2,4,6-Tris-[4-(4-hydroxy-3,5-di-tert.-butylbenzylcarbonyloxy)-2-thiabutyl]-phenol
2,4,6-Tris-[4-(4-hydroxy-3,5-di-tert.-butylphenyl)-2-thiabutyl]-phenol.

Die Herstellung der Verbindungen der Formel I erfolgt nach an sich bekannten Verfahren. So setzt man beispielsweise in Analogie zur Beschreibung in der GB-PS 1 184 533 einen Molanteil Phenol mit drei Molanteilen Formaldehyd und den entsprechenden Mengen geeignet substituierter Merkaptane um. Es lassen sich auch Gemische unterschiedlicher Merkaptane verwenden.

Die Reaktion lässt sich in An- oder in Abwesenheit eines organischen Lösungsmittels in Gegenwart eines basischen Katalysators bei Temperaturen zwischen 70°C und 150°C durchführen.

Geeignete Lösungsmittel sind Alkohole mit ein bis sechs Kohlenstoffatomen, wie beispielsweise Methanol, Ethanol, Propanol, Butanol, Pentanol oder Hexanol. Man kann aber auch Diole, Polyole sowie deren Ether verwenden, wie beispielsweise Glycol, Glycerin oder Polyethylenglykol. Die Reaktion lässt sich auch in polaren aprotischen Lösungsmitteln, wie beispielsweise Dimethylformamid oder Dimethylsulfoxid durchführen, oder es können hochsiedende aromatische oder aliphatische gegebenenfalls chlorierte Kohlenwasserstoffe wie beispielsweise Toluol, Ligroin oder Chlorbenzol eingesetzt werden. Als basische Katalysatoren verwendet man beispielsweise organische Basen, wie Dialkylamine oder Trialkylamine, oder man nimmt anorganische Basen, wie Hydroxide, vorzugsweise Alkalihydroxide. Anorganische Basen setzt man allerdings bevorzugt nur dann ein, wenn die Reaktanden keine hydrolisierbaren Gruppen wie beispielsweise Ester- oder Amidgruppen enthalten.

Anstelle des Formaldehyds können auch Verbindungen, die unter Reaktionsbedingungen Formaldehyd bilden, eingesetzt werden. Dazu zählen beispielsweise Paraformaldehyd oder Hexamethylentetramin.

Das Reaktionsgemisch wird wäherednd 5 bis 40 Stunden in einer Stickstoffatmosphäre unter Rückfluss erhitzt.

Nach dem Abkühlen auf Raumtemperatur wird die organische Phase mit einem geeigneten Lösungsmittel, beispielsweise mit Toluol, Chloroform, Methylenchlorid, Ether oder Methylisobutylketon, verdünnt. Anschliessend wird mit wässriger Säure neutral gewaschen. Dazu lässt sich beispielsweise Essigsäure einsetzen, aber es kann auch jede beliebige andere Säure, beispielsweise eine Mineralsäure genommen werden.

Die organische Phase wird nach der üblichen Abtrennung im Vakuum konzentriert und der Eindampfrückstand gegebenenfalls weiter gereinigt, beispielsweise durch Umkristallisieren, durch Säulenchromatographie oder durch Filtration über eine kurze Kieselgelsäule.

Die Verbindungen der Formel I lassen sich aber auch in Analogie zu dem in der US-PS 4 091 037 beschriebenen Verfahren durch Umsetzung einer geeigneten Trismannichbase und mindestens der stöchiometrischen Menge eines geeigneten Merkaptans oder einer Mischung geeigneter Merkaptane in An- oder Abwesenheit eines geeigneten organischen Lösungsmittels unter einer Stickstoffatmosphäre synthetisieren. Die Reaktionstemperatur liegt zwischen 100°C und 160°C, die Reaktionsdauer beträgt 10 bis 40 Stunden. Es eignen sich die gleichen organischen Lösungsmittel wie für die oben beschriebene Variante. Die Reaktion kann durch Anlegen eines leichten Vakuums beschleunigt werden (0,1 bis 0,6 bar). Die Aufarbeitung erfolgt wie bereits weiter oben beschrieben.

Verbindungen der Formel I, worin $R^1$, $R^2$ oder $R^3$ die Gruppe $-CH_2CH_2-O-CO-R^{10}$ bedeuten, können vorzugsweise aus dem entsprechenden Alkohol-Derivat durch Veresterung mit Säure oder Säurechlorid, oder durch Umesterung mit einem Methyl- oder Ethylester auf bekannter Weise hergestellt werden.

Die Erfindung betrifft weiterhin Zusammensetzungen enthaltend ein gegen thermischen, oxidativen oder strahlungsinduzierten Abbau empfindliches organisches Material und mindestens eine Verbindung der Formel I.

In einer bevorzugten Ausführungsform enthalten die erfindungsgemässen Zusammensetzungen Gemische von Verbindungen der Formel I.

Bevorzugt werden weiterhin Zusammensetzungen, worin das organische Material ein Polymeres, insbesondere ein Elastomers, ein Polyolefin oder ein Styrolpolymeres, bedeutet. Als Elastomere werden insbesondere bevorzugt:

Polydiene, wie beispielsweise Polybutadien, Polyisopren oder Polychloropren; Blockpolymere, wie beispielsweise Styrol/Butadien/Styrol, Styrol/Isopren/Styrol oder Styrol/Ethylen-Propylen/Styroltypen; sowie Acrylnitril/Butadien Polymere.

Diese Polymere können auch in Form von Latices vorliegen und können als solche stabilisiert sein.

Ebenfalls bevorzugt werden Zusammensetzungen, worin das organische Material ein synthetischer Schmierstoff oder ein Schmierstoff auf Mineralölbasis ist.

Die in Frage kommenden Schmierstoffe sind dem Fachmann geläufig und z.B. im «Schmiermittel Taschenbuch (Hüthig Verlag, Heidelberg, 1974)» beschrieben.

Ein weiterer Gegenstand der Erfindung ist die Verwendung von Verbindungen der Formel I als Stabilisatoren für organisches Material gegen dessen Schädigung durch Einwirkung von Sauerstoff, Wärme, Licht und energiereiche Strahlung.

Bevorzugt ist die Verwendung der Verbindungen als Antioxidantien in organischen Polymeren besonders in Polyolefinen, Styrolpolymeren oder in Elastomeren, oder die Verwendung in Mineralölen oder synthetischen Ölen.

Die erfindungsgemässen Verbindungen eignen sich auch als EP/AW Zusätze für Schmierstoffe oder Metallbearbeitungsflüssigkeiten.

Weitere Beispiele für organisches Material, welches mit den erfindungsgemässen Verbindungen vorteilhaft stabilisiert werden kann, sind:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopropen oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Isomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z.B. mit Polyethylenglykol, Polypropylenglykol oder Polytetramethylenglykol.

16. Polyharnstoffe, Polyamide, Polyamid-imide und Polybenzimidazole.

17. Polyester, die sich Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyesteracrylaten.

24. Alkydharze, Polyesterharze und Acrylharze, die mit Melaminharzen, Harnstoffharzen, Polyisocyanaten, oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z.B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellithate), sowie Abmischungen synthetischer Ester mit Mineralöl in beliebigen Gewichtsverhältnissen, wie sie z.B. als Spinnpräparationen Anwendung finden, sowie deren wässrigen Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die Stabilisatoren werden den Kunststoffen bzw. den Schmiermitteln in einer Konzentration von 0.01-10 gew.-% berechnet auf das zu stabilisierende Material zugesetzt. Vorzugsweise werden 0,05 bis 5,0 Gew.-%, besonders bevorzugt 0,1 bis 2,0 Gew.-% der Verbindungen, berechnet auf das zu stabilisierende Material, in dieses eingearbeitet.

Die Einarbeitung kann beispielsweise durch Einmischen der Substanzen der Formel I und gegebenenfalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels erfolgen. Die neuen Verbindungen können auch in Form eines Masterbatches, der diese Verbindungen beispielsweise in einer Konzentration von 2,5 bis 25 gew.-% enthält, den zu stabilisierenden Kunststoffen zugesetzt werden.

Im Falle von vernetztbarem Polyethylen werden die Verbindungen vor der Vernetzung beigefügt.

Die so stabilisierten Materialien können in verschiedenster Form angewendet werden, z.B. als Folien, Faser, Bändchen, Formmassen, Profile oder als Bindemittel für Lacke, Klebstoffe oder Kitte.

In der Praxis können die Phenole der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z.B. weitere Antioxidantien, Metallpassivatoren, Rostinhibitoren, Viskositäts-Index Verbesserer, Stockpunkterniedriger, Dispergiermittel/Tenside und Verschleissschutzadditive.

Als weitere Additive, mit denen zusammen die erfindungsgemäss verwendeten Stabilisatoren eingesetzt werden können, sind beispielsweise zu nennen:

*1. Antioxidantien*

*1.1. Alkylierte Monophenole*

2,6-Di-tert.-butyl-4-methylphenol
2-Tert.-butyl-4,6-dimethylphenol
2,6-Di-tert.-butyl-4-ethylphenol
2,6-Di-tert.-butyl-4-n-butylphenol
2,6-Di-tert.-butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.-butyl-4-methoxymethylphenol

*1.2. Alkylierte Hydrochinone*

2,6-Di-tert.-butyl-4-methoxyphenol
2,5-Di-tert.-butyl-hydrochinon
2,5-Di-tert.-amyl-hydrochinon
2,6-Diphenyl-4-octadecyloxyphenol

*1.3. Hydroxylierte Thiodiphenylether*

2,2'-Thio-bis(6-tert.-butyl-4-methylphenol)
2,2'-Thio-bis-(4-octylphenol)
4,4'-Thio-bis-(6-tert.-butyl-3-methylphenol)
4,4'-Thio-bis-(6-tert.-butyl-2-methylphenol)

*1.4. Alkyliden-Bisphenole*

2,2'-Methylen-bis-(6- tert.-butyl-4-methylphenol)
2,2'-Methylen-bis-(6-tert.-butyl-4-ethylphenol)
2,2'-Methylen-bis-[4-methyl-6-α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.-butylphenol)
2,2'-Ethyliden-bis-(4,6-di-tert.-butylphenol)
2,2'-Ethyliden-bis-(6-tert.-butyl-4-isobutylphenol)
2,2'-Methylen-bis-[6-(α-methylbenzyl)-4-nonylphenol]
2,2'-Methylen-bis-[6-α,α-dimethylbenzyl)-4-nonylphenol]
4,4'-Methylen-bis-(2,6-di-tert.-butylphenol)
4,4'-Methylen-bis-(6-tert.-butyl-2-methylphenol)
1,1-Bis-(5-tert.-butyl-4-hydroxy-2-methylphenyl)-butan
2,6-Di-(3-tert.-butyl-5-methyl-2-hydroxybenzyl)-4-methylphenol
1,1,3-Tris-(5-tert.-butyl-4-hydroxy-2-methylphenyl)-butan
1,1-Bis-(5-tert.-butyl-4-hydroxy-2-methylphenyl)-3-n-dodecylmercaptobutan
Ethylenglycol-bis-[3,3-bis-(3'-tert.-butyl-4'-hydroxyphenyl)-butyrat]
Di-(3-tert.-butyl-4-hydroxy-5-methylphenyl)-dicyclopentadien
Di-[2-(3'-tert.-butyl-2'-hydroxy-5'-methyl-benzyl)-6-tert.-butyl-4-methyl-phenyl]-terephthalat.

*1.5. Benzylverbindungen*

1,3,5-Tri-(3,5-di-tert.-butyl-4-hydroxybenzyl)-2,4,6-trimethylbenzol
Di-(3,5-di-tert.-butyl-4-hydroxybenzyl)-sulfid
3,5-Di-tert.-butyl-4-hydroxybenzyl-mercaptoessigsäure-isooctylester
Bis-(4-tert.-butyl-3-hydroxy-2,6-dimethylbenzyl)-dithiol-terephthalat
1,3,5-Tris-(3,5-di-tert.-butyl-4-hydroxybenzyl)-isocyanurat
1,3,5-Tris-(4-tert.-butyl-3-hydroxy-2,6-dimethylbenzyl)-isocyanurat
3,5-Di-tert.-butyl-4-hydroxybenzyl-phosphonsäure-dioctadecylester
3,5-Di-tert.-butyl-4-hydroxybenzyl-phosphonsäure-monoethylester,
Calcium-salz.

*1.6. Acylaminophenole*

4-Hydroxy-laurinsäureanilid
4-hydroxy-stearinsäureanilid
2,4-Bis-octylmercapto-6-(3,5-di-tert.-butyl-4-hydroxyanilino)-s-triazin
N-(3,5-di-tert.-butyl-4-hydroxyphenyl)-carbaminsäureoctylester.

*1.7. Ester der β-(3,5-Di-tert.-butyl-4-hydroxyphenyl)-propionsäure* mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglykol | Tris-hydroxyethyl-isocyanurat |
| Thiodiethylenglycol | Di-hydroxyethyl-oxalsäure-diamid |

*1.8. Ester der β-(5-tert.-butyl-4-hydroxy-3-methylphenyl)-propionsäure* mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglykol | Tris-hydroxyethyl-isocyanurat |
| Thiodiethylenglycol | Di-hydroxyethyl-oxalsäure-diamid |

*1.9. Amide der β-(3,5-Di-tert.-butyl-4-hydroxyphenyl)-propionsäure,* wie z.B.

N,N'-Di-(3,5-di-tert.-butyl-4-hydroxyphenylpropionyl)-hexamethylendiamin

N,N'-Di-(3,5-di-tert.-butyl-4-hydroxyphenylpropionyl)-trimethylendiamin
N,N'-Di-(3,5-di-tert.-butyl-4-hydroxyphenylpropionyl)-hydrazin

*2. UV-Absorber und Lichtschutzmittel*

*2.1. 2-(2'-Hydroxyphenyl)-benztriazole,* wie z.B. das 5'-Methyl-, 3',5'-Di-tert.-butyl-, 5'-Tert.-butyl-, 5'-(1,1,3,3-Tetramethylbutyl)-, 5-Chlor-3',5'-di-tert.-butyl-, 5-Chlor-3'-tert.-butyl-5'-methyl-, 3'-sec.-Butyl-5'-tert.-butyl-, 4'-Octoxy-, 3',5'-Di-tert.-amyl-, 3',5'-Bis-(α,α-dimethylbenzyl)-Derivat.

*2.2. 2-Hydroxybenzophenone,* wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

*2.3. Ester von gegebenenfalls substituierten Benzoesäuren,* wie z.B. 4-Tert.-butyl-phenylsalicylat, Phenylsalicylat, Octylphenylsalicylat, Dibenzoylresorcin, Bis-(4-tert.-butylbenzoyl)-resorcin, Benzoylresorcin, 3,5-Di-tert.-butyl-4-hydroxybenzoesäure-2,4-di-tert.-butylphenylester, 3,5-Di-tert.butyl-4-hydroxybenzoesäurehexadecylester.

*2.4. Acrylate,* wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

*2.5. Nickelverbindungen,* wie z.B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.-butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

*2.6. Sterisch gehinderte Amine,* wie z.B. Bis-(2,2,6,6-tetramethylpiperidyl)-sebacat
Bis-(1,2,2,6,6-pentamethylpiperidyl)-sebacat
n-Butyl-3,5-di-tert.-butyl-4-hydroxybenzyl-malonsäure-bis-(1,2,2,6,6-pentamethylpiperidyl)-ester, Kondensationsprodukt aus 1-Hydroxyethyl-2,2,-6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.-Octylamino-2,6-dichlor-1,3,5-s-triazin,
Tris-(2,2,6,6-tetramethyl-4-piperidyl)-nitrilotriacetat,
Tetrakis-(2,2,6,6-tetramethyl-4-piperidyl)-1,2,3,4-butantetracarbonsäureester,
1,1'-(1,2-Ethandiyl)-bis-(3,3,5,5-tetramethyl)-piperazinon).

*2.7. Oxalsäurediamide,* wie z.B. 4,4'-Di-octyloxyoxanilid, 2,2'-Di-octyloxy-5,5'-di-tert.-butyl-oxanilid, 2,2'-Di-dodecyloxy-5,5'-di-tert.-butyl-oxanilid, 2-Ethoxy-2'-ethyl-oxanilid, N,N'-Bis-(3-dimethylaminopropyl)-oxalamid, 2-Ethoxy-5-tert.-butyl-2'-ethyl-oxanilid und dessen Gemisch mit 2-Ethoxy-2'-ethyl-5,4'-di-tert.-butyl-oxanilid, Gemische von ortho- und para-Methoxy- sowie von o- und p-Ethoxydi-substituierte Oxaniliden.

*3. Metalldesaktivatoren,* wie z.B. N,N'-Diphenyloxalsäurediamid, N-Salicylal-N'-salicyloylhydrazin, N,N'-Bis-salicyloylhydrazin, N,N'-Bis-(3,5-di-tert.-butyl-4-hydroxyphenylpropionyl)-hydrazin, 3-Salicyloylamino-1,2,4-triazol, Bis-benzyliden-oxalsäuredihydrazid.

*4. Phosphite und Phosphonite,* wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.-butylphenyl)-phosphit, Diisodecylpentaerythrit-diphosphit, Di-(2,4-di-tert.-butylphenyl)-pentaerythritdiphosphit, Tristearyl-sorbit-triphosphit, Tetrakis-(2,4-di-tert.-butylphenyl)-4,4'-biphenylen-diphosphonit.

*5. Peroxidzerstörende Verbindungen,* wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

*6. Polyamidstabilisatoren,* wie z.B. Kupfersalze in Kombination mit Jodiden und/oder Phosphorverbindungen und Salze des zweiwertigen Mangans.

*7. Basische Co-Stabilisatoren,* wie z.B. Melamin, Polyvinylpyrrolidon, Dicyanamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

*8. Nukleierungsmittel,* wie z.B. 4-tert.-Butylbenzoesäure, Adipinsäure, Diphenylessigsäure.

*9. Füllstoffe und Verstärkungsmittel,* wie z.B. Calciumcarbonat, Silikate, Glasfasern, Asbest, Talk, Kaolin, Glimmer, Bariumsulfat, Metalloxide und -hydroxide, Russ, Graphit.

*10. Sonstige Zusätze,* wie z.B. Weichmacher, Gleitmittel, Emulgatoren, Pigmente, Optische Aufheller, Flammschutzmittel, Antistatika, Treibmittel.

*11. Aminische Antioxidantien:*
N,N'-Di-isopropyl-p-phenylendiamin
N,N'-Di-sec.-butyl-p-phenylendiamin
N,N'-Bis-(1,4-dimethyl-pentyl)-p-phenylendiamin
N,N'-Bis-(1-ethyl-3-methyl-pentyl)-p-phenylendiamin
N,N'-Bis-(1-methyl-heptyl)-p-phenylendiamin
N,N'-Dicyclohexyl-p-phenyldiamin
N,N'-Diphenyl-p-phenyldiamin
N,N'-Di-(naphthyl-2)-p-phenyldiamin

N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec.-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert.-butyl-4-dimethylamino-methyl-phenol
2,4'-Diamino-diphenylmethan
4,4'-Diamino-diphenylmethan
N,N,N,N'-Tetramethyl-4,4'-diamino-diphenylmethan
1,2-Di-(phenylamino)-ethan
1,2-Di-[(2-methyl-phenyl)-amino]-ethan
1,3-Di-(phenylamino)-propan
(o-Tolyl)-biguanid
Di-[4-(1',3'-dimethyl-butyl)-phenyl]-amin.

*12. Metallpassivatoren:*
für Kupfer, z.B.
Benztriazol, Tetrahydrobenztriazol, 2-Mercaptobenzthiazol, 2,5-Dimercaptothiadiazol, Salicylidenpropylendiamin, Salze von Salicylaminoguanidin.

*13. Rost-Inhibitoren:*

a) Organische Säuren, ihre Ester, Metallsalze und Anhydride, z.B.:
N-Oleoyl-sarcosin, Sorbitan-mono-oleat, Bleinaphthenat, Dodecenylbernsteinsäure-anhydrid, Alkenylbernsteinsäure-Halbester, 4-Nonylphenoxyessigsäure.

b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.

II. Heterocyclische Verbindungen z.B.: Substituierte Imidazoline und Oxazoline.

c) Phosphorhaltige Verbindungen, z.B.: Aminsalze von Phosphorsäurepartialestern.

d) Schwefelhaltige Verbindungen, z.B.: Barium-dinonylnaphathalin-sulfonate, Calciumpetroleum-sulfonate.

*14. Viskositätsindex-Verbesserer:*
Polymethacrylate, Vinylpyrrolidon/Methacrylat-Copolymere, Polybutene, Olefin-Copolymere, Styrol/Acrylat-Copolymere.

*15. Stockpunktniedriger:*
Polymethacrylat, alkylierte Naphthalinderivate.

*16. Dispergiermittel/Tenside:*
Polybutenylbernsteinsäure-imide, Polybutenylphosphonsäurederivate, basische Magnesium-, Calcium-, und Bariumsulfonate und -phenolate.

*17. Verschleissschutz-Additive:*
Schwefel und/oder Phosphor und/oder Halogen enthaltende Verbindungen, wie geschwefelte pflanzliche Öle, Zinkdialkylditiophosphate, Tritolylphosphat, chlorierte Paraffine, Alkyl- und Aryldisulfide.

*Herstellungsbeispiel*

*Beispiel 1*

26,5 g (0,1 Mol) 2,4,6-Tris-(dimethylaminomethyl)-phenol und 61,6 g (0,3 Mol) Thioglykolsäure-2-ethyl-hexylester werden bei Raumtemperatur gemischt und während 10 Stunden unter einem leichten Stickstoffstrom auf 140°C erwärmt. Danach wird bei 80°C während 2 Stunden im Hochvakuum (0,7 mbar) vollständig entgast. Der Rückstand wird durch Säulenchromatographie weiter gereinigt 2,4,6-Tris-[(2-ethylhexyloxycarbonyl)-methylthiomethyl]-phenol wird als klares, gelbliches Öl erhalten: Ber. S 12,94%, Gef. S 13,07% (charakteristische $^{1}$H-NMR-Signale in Tabelle 1).

*Beispiel 2*

Die gleiche Verbindung wird erhalten, wenn 9,4 g Phenol, 61,3 g Thioglykolsäure-2-ethylhexylester, 9 g Paraformaldehyd, 30 ml Dimethylformamid sowie 2,4 g Dibutylamin unter leichtem Stickstoffstrom während 8 Stunden auf 130° erhitzt, am Rotationsverdampfer vom Lösungsmittel befreit und anschliessend wie in Beispiel 1 beschrieben weiter aufgearbeitet werden.

*Beispiel 3*

Verfährt man wie in Beispiel 1 beschrieben, ausser dass man anstelle von Thioglykolsäure-2-ethylhexylester die entsprechende Menge 3-Mercaptopropionsäure-2-ethylhexylester einsetzt, so erhält man 2,4,6-Tris-([2-(2-ethylhexyl)oxycarbonylethyl]thiomethyl)-phenol als gelbliche Flüssigkeit. Ber. S 12,25%, Gef. S 12,38% (charakteristische $^{1}$H-NMR-Signale in Tabelle 1).

*Beispiel 4*

Verfährt man wie in Beispiel 1 beschrieben, ausser dass man anstelle von Thioglykolsäure-2-ethylhexylester die entsprechende Menge Thioglykolsäurestearylester einsetzt, so erhält man 2,4,6-Tris-stearyloxy-carbonylmethylthiomethyl)-phenol vom Smp. 70-71°C. Ber. S 8,26%, Gef. S 8,34% (charakteristische $^{1}$H-NMR-Signale in Tabelle 1).

*Beispiel 5*

Verfährt man wie in Beispiel 1 beschrieben, ausser dass man anstelle von Thioglykolsäure-2-ethylhexylester die entsprechende Menge 2-Mercaptoethanol einsetzt, so erhält man 2,4,6-Tris-(2-hydroxyethylthiomethyl)-phenol als gelbliches Öl. Ber. S 26,38%, gef. S 26,40% (charakteristische $^{1}$H-NMR-Signale in Tabelle 1).

Tabelle 1

Charakteristische $^1$H-NMR-Signale der beanspruchten Verbindungen [1)]

| Beispiel Nr. | $R^1 = R^2 = R^3$ | Signal Aryl-$CH_2$-S ortho-$CH_2$ (ppm) | para-$CH_2$ (ppm) | Gerätetyp |
|---|---|---|---|---|
| 1 | $CH_2COOCH_2CH(C_2H_5)$-$(CH_2)_3$-$CH_3$ | 3,80 | 3,67 | (a) |
| 3 | $CH_2CH_2COOCH_2CH(C_2H_5)$-$(CH_3)$-$CH_3$ | 3,72 | 3,60 | (a) |
| 4 | $CH_2COO(CH_2)_{17}$-$CH_3$ | 3,80 | 3,67 | (a) |
| 5 | $CH_2CH_2OH$ | 3,85 | 3,70 | (b)[2)] |

(a) 60 MHz [1)] aufgenommen in $CDCl_3$, mit TMS als Standard
(B) 100 MHz [2)] in $d_6$-Aceton

*Anwendungsbeispiele*

*Beispiel 6*

*TFOUT-Test* (Thin-Film Oxygen Uptake Test)

Dieser Test- ist eine modifizierte Version des «Rotary Bomb Oxidationstests für Mineralöle» (ASTM D 2272). Er wird genau beschrieben in «C.S. Ku und S.M. Hsu, A Thin-Film Oxygen Uptake Test for the Evaluation of Automotive Crankcase Lubricants, *Lubrication Engineering*, Vol. 40 (2), 75-83 (1984)». Als Testöl dient ein Motorenöl auf Mineralölbasis, welches die Hälfte der üblichen Menge an Zinkdithiophosphat (0,75%; Zinkgehalt 0,06%, bezogen auf das Motorenöl) enthält.

Die in Beispiel 1 hergestellte Verbindung wird im beschriebenen Motorenöl in Gegenwart von 2% Wasser, einer flüssigen oxidierten, nitrierten Fraktion eines Motorenbenzins als Katalysator (4% Einsatzkonzentration), eines flüssigen Metallnaphthenates als weiterer Katalysator (4% Einsatzkonzentration; Wasser und die beiden flüssigen Katalysatorsubstanzen sind unter der No. Standard Reference Material 1817 vom National Bureau of Standards (NBS) mit Analysen-Zertifikat geliefert worden) getestet. Der Versuch ist bei einem deutlichen Knick des Druck/Zeit-Diagramms beendet. Die in der untenstehenden Tabelle angegebenen Resultate bedeuten die Zeit (in Minuten) bis zum Knick des Druck/Zeit-Diagramms.

Lange Zeiten entsprechen guter Stabilisatorwirksamkeit. Konzentration des Stabilisators: 0,5 Gew.-%, bezogen auf das Öl.

| Stabilisator | Minuten bis deutlicher Druckabfall |
|---|---|
| keiner | 76 |
| Beispiel 1 | 128 |

**Patentansprüche**

1. Verbindungen der Formel I

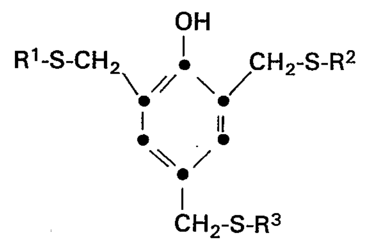

(I)

worin $R^1$, $R^2$ und $R^3$ unabhängig voneinander durch ein oder zwei Hydroxygruppen substituiertes $C_2$-$C_{20}$ Alkyl, -$C(R^4R^5)$-$(CHR^6)_n$-W, -$(CH_2)_2$-$OCOR^{10}$ oder ein Rest

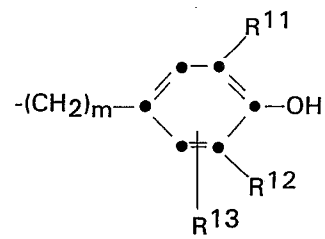

bedeuten, worin $R^4$, $R^5$ und $R^6$ unabhängig voneinander Wasserstoff, $C_1$-$C_6$ Alkyl, Phenyl oder Cyclohexyl sind, W -$COR^7$, -$COOR^8$, -$CON(R^9R^{15})$ oder -CN ist, wobei $R^7$ Wasserstoff, $C_1$-$C_{20}$ Alkyl, $C_5$-$C_{12}$ Cycloalkyl, Phenyl, 1-Naphthyl, 2-Naphthyl, $C_7$-$C_{14}$ Aralkyl oder $C_7$-$C_{14}$ Alkaryl bedeutet, $R^8$ $C_1$-$C_{20}$ Alkyl, $C_5$-$C_{12}$ Cycloalkyl, Phenyl, 1- oder 2-Naphthyl, $C_7$-$C_{14}$ Aralkyl, $C_7$-$C_{14}$ Alkaryl oder durch eine Hydroxy- oder Cyangruppe substituiertes $C_2$-$C_{20}$ Alkyl ist, oder $C_2$-$C_{12}$ Alkenyl, $C_2$-$C_{12}$ Alkinyl, durch ein oder zwei -$NO_2$, -Cl, -Br, -$OCH_3$ oder -$COOR^{14}$ substituiertes Phenyl bedeutet oder ein Rest

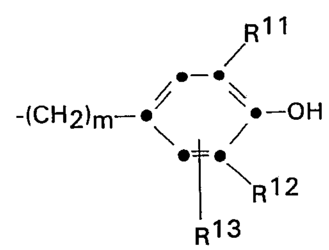

ist, oder durch ein bis fünf -O-, -S-, $-N(CH_3)-$, $-N(C_2H_5)-$ oder $-SO_2-$ unterbrochenes gegebenenfalls mit einer Hydroxygruppe substituiertes $C_3$-$C_{20}$ Alkyl bedeutet, wobei gegebenenfalls mehrfach auftretende Heteroatome durch mindestens eine Methylengruppe getrennt sein müssen, $R^9$ eine der Bedeutungen von $R^8$ besitzt oder zusätzlich Wasserstoff bedeutet, $R^{15}$ eine der Bedeutungen von $R^9$ besitzt oder worin $R^9$ und $R^{15}$ zusammen mit dem gemeinsamen Stickstoffatom einen fünf-, sechs- oder siebengliedrigen heterocyclischen Ring bilden, der gegebenenfalls noch ein weiteres Heteroatom enthalten kann, $R^{10}$ $C_1$-$C_{20}$ Alkyl, $C_5$-$C_{12}$ Cycloalkyl, Phenyl, 1- oder 2-Naphthyl, $C_7$-$C_{14}$ Aralkyl, $C_7$-$C_{14}$ Alkaryl oder einen Rest

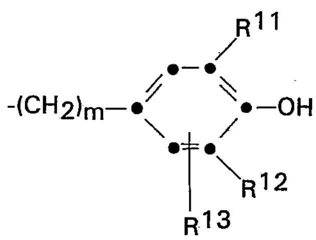

bedeutet, m 0, 1 oder 2 ist, n für 0 oder 1 steht, $R^{11}$, $R^{12}$ oder $R^{13}$ unabhängig voneinander Wasserstoff, $C_1$-$C_{20}$ Alkyl, Cyclohexyl oder Phenyl sind, und schliesslich $R^{14}$ $C_1$-$C_6$ Alkyl, Phenyl, Cyclohexyl, Benzyl oder Tolyl ist.

2. Verbindungen der Formel I gemäss Anspruch 1, worin $R^1$, $R^2$ und $R^3$ unabhängig voneinander 2-Hydroxyethyl, 2-Hydroxypropyl oder 2,3-Dihydroxypropyl bedeuten.

3. Verbindungen der Formel I gemäss Anspruch 1, worin $R^1$, $R^2$ und $R^3$ unabhängig voneinander $-C(R^4R^5)-(CHR^6)_n-W$, $-(CH_2)_2-OCOR^{10}$ oder einen Rest

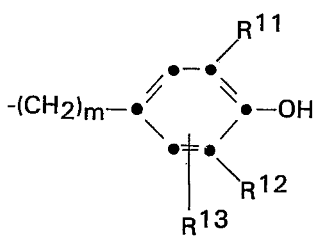

bedeuten und die Symbole $R^4$, $R^5$, $R^6$, $R^{10}$, $R^{11}$, $R^{12}$, $R^{13}$, W, m und n die in Anspruch 1 genannte Bedeutung haben.

4. Verbindungen der Formel I gemäss Anspruch 3, worin $R^4$, $R^5$ und $R^6$ unabhängig voneinander Wasserstoff oder Methyl bedeuten, $R^7$ $C_1$-$C_{12}$ Alkyl, $C_5$-$C_9$-Cycloalkyl, Phenyl, Benzyl oder Tolyl ist, $R^8$, $R^9$ und $R^{15}$ unabhängig voneinander die gleiche Bedeutung wie $R^7$ besitzen oder zusätzlich 2-Hydroxyethyl, 2-Hydroxypropyl, 2-Cyanethyl, Allyl, Propargyl, durch ein -Cl, $-COOCH_3$ oder $-OCH_3$ substituiertes Phenyl oder ein Rest

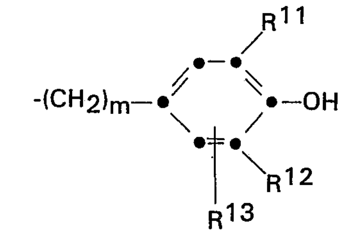

bedeuten, oder $R^8$, $R^9$ und $R^{15}$ durch ein bis fünf -O- unterbrochenes gegebenenfalls mit einer Hydroxygruppe substituiertes $C_3$-$C_{12}$ Alkyl darstellen, wobei gegebenenfalls mehrfach auftretende Sauerstoffatome durch mindestens eine Methylengruppe getrennt sein müssen, oder $R^9$ und $R^{15}$ zusammen mit dem gemeinsamen Stickstoffatom einen Pyrrol-, Piperidin-, Pyrrolidin- oder Morpholinring bilden, $R^{10}$ $C_1$-$C_{12}$ Alkyl, $C_5$-$C_9$ Cycloalkyl, Phenyl $C_7$-$C_9$ Aralkyl oder ein Rest

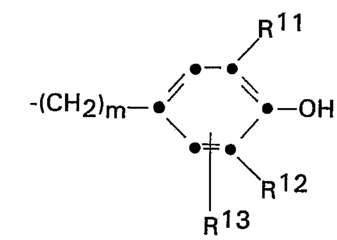

bedeutet, m 0, 1 oder 2 ist und schliesslich $R^{11}$, $R^{12}$ und $R^{13}$ unabhängig voneinander Wasserstoff oder $C_1$-$C_{12}$ Alkyl sind.

5. Verbindungen der Formel I gemäss Anspruch 1, worin $R^4$, $R^5$ und $R^6$ unabhängig voneinander Wasserstoff oder Methyl sind, W $-COOR^8$ oder $-CON(R^9R^{15})$ ist, wobei $R^8$, $R^9$ und $R^{15}$ unabhängig voneinander $C_1$-$C_{12}$ Alkyl, Cycloalkyl, Phenyl, Benzyl, Tolyl oder einen Rest

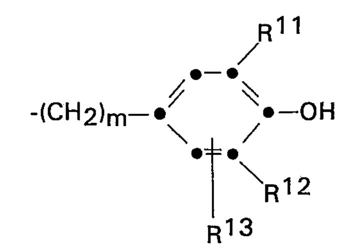

bedeuten, oder worin $R^8$, $R^9$ und $R^{15}$ durch ein bis fünf -O- unterbrochenes gegebenenfalls durch eine Hydroxygruppe substituiertes $C_3$-$C_{12}$ Alkyl sind, wobei gegebenenfalls mehrfach auftretende Sauerstoffatome durch mindestens eine Methylengruppe getrennt sein müssen, $R^{10}$ $C_1$-$C_{12}$ Alkyl, Cyclohexyl, Phenyl, Benzyl oder ein Rest

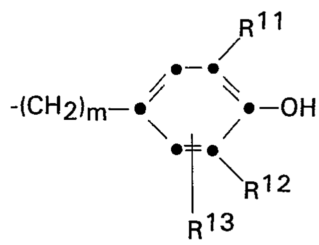

bedeutet, m 0, 1 oder 2 ist, und $R^{11}$, $R^{12}$ und $R^{13}$ unabhängig voneinander für Wasserstoff oder tert.-Butyl stehen.

6. Verbindungen der Formel I gemäss Anspruch 1, worin $R^1$, $R^2$ und $R^3$ $-C(R^4R^5)-(CHR^6)_n-W$ bedeuten, worin $R^4$, $R^5$ und $R^6$ unabhängig voneinander Wasserstoff oder Methyl sind, W $-COOR^8$ oder $-CON(R^9R^{15})$ ist, $R^8$, $R^9$ und $R^{15}$ unabhängig voneinander $C_1$-$C_{12}$ Alkyl, Cyclohexyl, Phenyl, Benzyl, Tolyl oder ein Rest

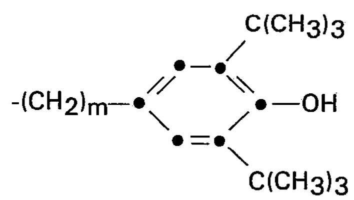

bedeuten, oder 2-Hydroxyethyl, 2-Hydroxypropyl, 3-Oxabutyl oder ein Rest $-(CH_2-CH_2-O)_p-CH_2-CH_2-OH$ sind, wobei p 1, 2 oder 3 ist, und m und n die in Anspruch 1 genannte Bedeutung haben oder worin $R^9$ und $R^{15}$ zusammen mit dem gemeinsamen Stickstoffatom einen Piperidin- oder Morpholinring bilden.

7. Verbindungen der Formel I gemäss Anspruch 1, worin $R^1$, $R^2$ und $R^3$ $-(CH_2)_2-OCOR^{10}$ sind, $R^{10}$ $C_1$-$C_{12}$ Alkyl, Cyclohexyl, Phenyl, Benzyl oder ein Rest

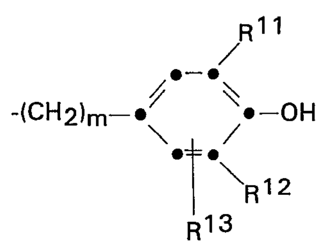

ist, worin $R^{11}$, $R^{12}$ und $R^{13}$ unabhängig voneinander Wasserstoff oder tert.-Butyl sind und m 0, 1 oder 2 ist.

8. Verbindungen der Formel I gemäss Anspruch 1, worin $R^1$, $R^2$ und $R^3$ Reste der Formel

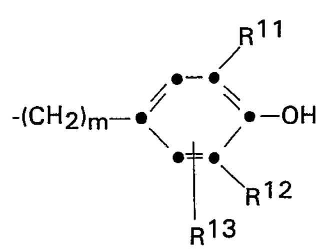

sind, und worin $R^{11}$, $R^{12}$ und $R^{13}$ unabhängig voneinander Wasserstoff oder tert.-Butyl sind und m 9, 1 oder 2 ist.

9. Eine Verbindung der Formel I gemäss Anspruch 1, worin $R^1$, $R^2$ und $R^3$ den Rest

$-CH_2-COO-CH_2-CH-(CH_2)_3-CH_3$
$\qquad\qquad\qquad\quad |$
$\qquad\qquad\qquad CH_2CH_3$

bedeuten.

10. Zusammensetzungen enthaltend ein gegen thermischen. oxidativen oder strahlungsinduzierten Abbau empfindliches organisches Material und mindestens eine Verbindung der Formel I gemäss Anspruch 1.

11. Zusammensetzung gemäss Anspruch 9, worin das organische Material ein Polymeres oder ein Schmierstoff ist.

12. Zusammensetzung gemäss Anspruch 9, worin das organische Material ein Polyolefin, ein Elastomeres oder ein Styrolpolymeres ist.

13. Zusammensetzung gemäss Anspruch, worin das organische Material ein Mineralöl oder ein synthetischer Schmierstoff ist.

14. Verwendung von Verbindungen der Formel I gemäss Anspruch 1 als Stabilisatoren für organisches Material gegen dessen Schädigung durch Einwirkung von Sauerstoff, Wärme, Licht oder energiereiche Strahlung.

## Claims

1. A compound of the formula I

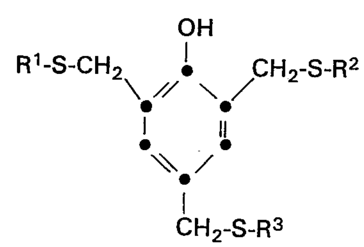

(I)

in which $R^1$, $R^2$ and $R^3$ independently of one another are $C_2$-$C_{20}$-alkyl, $-C(R^4R^5)-(CHR^6)_n-W$ or $-(CH_2)_2-OCOR^{10}$ which is substituted by one or two hydroxyl groups or are a radical

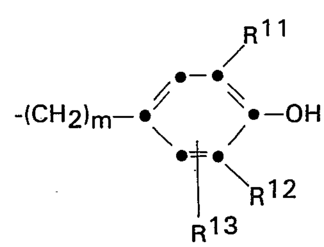

in which $R^4$, $R^5$ and $R^6$ independently of one another are hydrogen, $C_1$-$C_6$-alkyl, phenyl or cyclohexyl, W is $-COR^7$, $-COOR^8$, $-CON(R^9R^{15})$ or -CN, $R^7$ being hydrogen, $C_1$-$C_{20}$-alkyl, $C_5$-$C_{12}$-cycloalkyl, phenyl,

1-naphthyl, 2-naphthyl, $C_7$-$C_{14}$-aralkyl or $C_7$-$C_{14}$-alkaryl, $R^8$ being $C_1$-$C_{20}$-alkyl, $C_5$-$C_{12}$-cycloalkyl, phenyl, 1-naphthyl, 2-naphthyl, $C_7$-$C_{14}$-aralkyl, $C_7$-$C_{14}$-alkaryl or $C_2$-$C_{20}$-alkyl which is substituted by a hydroxyl or cyano group, or $C_2$-$C_{12}$-alkenyl, $C_2$-$C_{12}$-alkinyl or phenyl which is substituted by one or two $-NO_2$, -Cl, -Br, $-OCH_3$ or $-COOR^{14}$ groups, or a radical

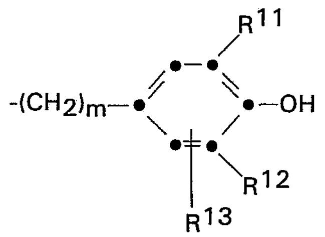

or $C_3$-$C_{20}$-alkyl which is interrupted by one to five -O-, -S-, $-N(CH_3)$-, $-N(C_2H_5)$- or $-SO_2$- groups and is unsubstituted or substituted by a hydroxyl group, it being necessary for any hetero-atoms occurring several times to be separated by at least one methylene group, $R^9$ having one of the meanings of $R^8$ or being additionally hydrogen, $R^{15}$ having one of the meanings of $R^9$, or $R^9$ and $R^{15}$, together with the common nitrogen atom, forming a 5-membered, 6-membered or 7-membered, heterocyclic ring which can if desired also contain a further hetero-atom, $R^{10}$ being $C_1$-$C_{20}$-alkyl, $C_5$-$C_{12}$-cycloalkyl, phenyl, 1-naphthyl, 2-naphthyl, $C_7$-$C_{14}$-aralkyl, $C_7$-$C_{14}$-alkaryl or a radical

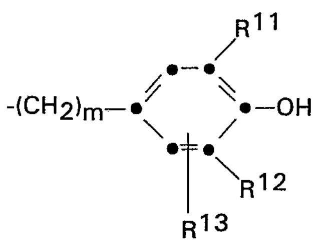

m being 0, 1 or 2, n being 0 or 1, $R^{11}$, $R^{12}$ or $R^{13}$ independently of one another being hydrogen, $C_1$-$C_{20}$-alkyl, cyclohexyl or phenyl and, finally, $R^{14}$ being $C_1$-$C_6$-alkyl, phenyl, cyclohexyl, benzyl or tolyl.

2. A compound of the formula I, according to claim 1, in which $R^1$, $R^2$ and $R^3$ independently of one another are 2-hydroxyethyl, 2-hydroxypropyl or 2,3-dihydroxypropyl.

3. A compound of the formula I, according to claim 1, in which $R^1$, $R^2$ and $R^3$ independently of one another are $-C(R^4R^5)-(CHR^6)_n-W$, $-(CH_2)_2-OCOR^{10}$ or a radical

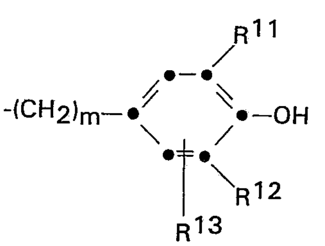

and the symbols $R^4$, $R^5$, $R^6$, $R^{10}$, $R^{11}$, $R^{12}$, $R^{13}$, W, m and n are as defined in claim 1.

4. A compound of the formula I, according to claim 3, in which $R^4$, $R^5$ and $R^6$ independently of one another are hydrogen or methyl, $R^7$ is $C_1$-$C_{12}$-alkyl, $C_5$-$C_9$-cycloalkyl, phenyl, benzyl or tolyl, $R^8$, $R^9$ and $R^{15}$ independently of one another have the same meaning as $R^7$ or additionally are 2-hydroxyethyl, 2-hydroxypropyl, 2-cyanoethyl, allyl, propargyl or phenyl which is substituted by -Cl, $-COOCH_3$ or $-OCH_3$ or are a radical

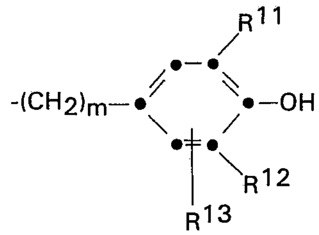

or $R^8$, $R^9$ and $R^{15}$ are $C_3$-$C_{12}$-alkyl which is interrupted by one to five -O- groups and is unsubstituted or substituted by a hydroxyl group, it being necessary for any oxygen atoms occurring several times to be separated by at least one methylene group, or $R^9$ and $R^{15}$, together with the common nitrogen atom, form a pyrrole, piperidine, pyrrolidine or morpholine ring, $R^{10}$ is $C_1$-$C_{12}$-alkyl, $C_5$-$C_9$-cycloalkyl, phenyl, $C_7$-$C_9$-aralkyl or a radical

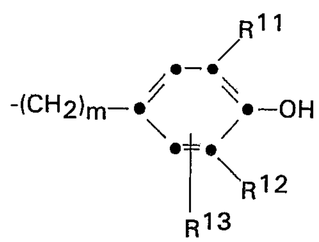

m is 0, 1 or 2 and, finally, $R^{11}$, $R^{12}$ and $R^{13}$ independently of one another are hydrogen or $C_1$-$C_{12}$-alkyl.

5. A compound of the formula I, according to claim 1, in which $R^4$, $R^5$ and $R^6$ independently of one another are hydrogen or methyl, W is $-COOR^8$ or $-CON(R^9R^{15})$, $R^8$, $R^9$ and $R^{15}$ independently of one another are $C_1$-$C_{12}$-alkyl, cycloalkyl, phenyl, benzyl, tolyl or a radical

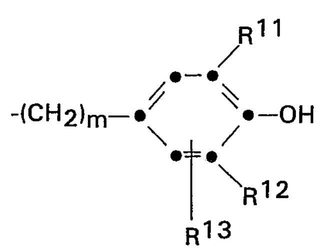

or in which $R^8$, $R^9$ and $R^{15}$ are $C_3$-$C_{12}$-alkyl which is interrupted by one to five -O- groups and is unsubstituted or substituted by a hydroxyl group, it being necessary for any oxygen atoms occurring several times to be separated by at least one methylene group, and in which $R^{10}$ is $C_1$-$C_{12}$-alkyl, cyclohexyl, phenyl, benzyl or a radical

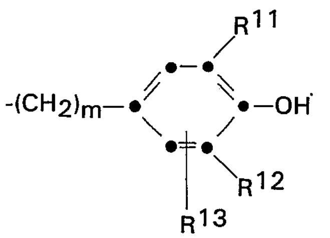

m is 0, 1 or 2 and $R^{11}$, $R^{12}$ and $R^{13}$ independently of one another are hydrogen or tert.-butyl.

6. A compound of the formula I, according to claim 1, in which $R^1$, $R^2$ and $R^3$ are $-C(R^4R^5)-(CHR^6)_n-W$, in which $R^4$, $R^5$ and $R^6$ independently of one another are hydrogen or methyl, W is $-COOR^8$ or $-CON(R^9R^{15})$, $R^8$, $R^9$ and $R^{15}$ independently of one another are $C_1$-$C_{12}$-alkyl, cyclohexyl, phenyl, benzyl, tolyl or a radical

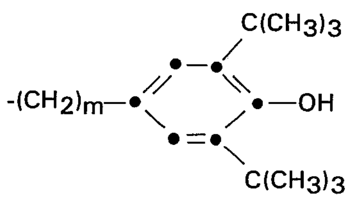

or are 2-hydroxyethyl, 2-hydroxypropyl, 3-oxabutyl or a radical $-(CH_2-CH_2-O)_p-CH_2-CH_2-OH$, p being 1, 2 or 3, and m and n are as defined in claim 1, or in which $R^9$ and $R^{15}$, together with the common nitrogen atom, form a piperidine or morpholine ring.

7. A compound of the formula I, according to claim 1, in which $R^1$, $R^2$ and $R^3$ are $-(CH_2)_2-OCOR^{10}$ and $R^{10}$ is $C_1$-$C_{12}$-alkyl, cyclohexyl, phenyl, benzyl or a radical

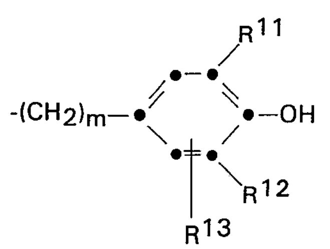

in which $R^{11}$, $R^{12}$ and $R^{13}$ independently of one another are hydrogen or tert.-butyl and m is 0, 1 or 2.

8. A compound of the formula 1, according to claim 1, in which $R^1$, $R^2$ and $R^3$ are radicals of the formula

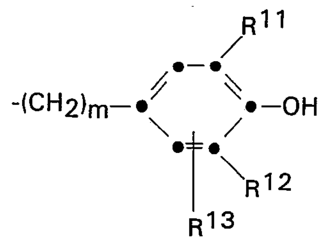

and in which $R^{11}$, $R^{12}$ and $R^{13}$ independently of one another are hydrogen or tert.-butyl and m is 0, 1 or 2.

9. A compound of the formula I, according to claim 1, in which $R^1$, $R^2$ and $R^3$ are the radical

$-CH_2-COO-CH_2-CH-(CH_2)_3-CH_3$
$\quad\quad\quad\quad\quad\quad\quad |$
$\quad\quad\quad\quad\quad\quad CH_2CH_3$

10. A composition containing an organic material which is sensitive to thermal, oxidative or radiation-induced degradation and at least one compound of the formula I according to claim 1.

11. A composition according to claim 9, in which the organic material is a polymer or a lubricant.

12. A composition according to claim 9, in which the organic material is a polyolefin, an elastomer or a styrene polymer.

13. A composition according to claim 9, in which the organic material is a mineral oil or a synthetic lubricant.

14. The use of a compound of the formula I according to claim 1 as a stabiliser for organic material against decomposition of the latter caused by the action of oxygen, heat, light or high-energy radiation.

**Revendications**

1. Composés répondant à la formule I:

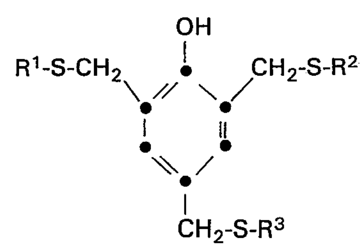

(I)

dans laquelle $R^1$, $R^2$ et $R^3$ représentent chacun, indépendamment les uns des autres, un radical alkyle $C_2$-$C_{20}$ porteur d'un ou de deux radicaux hydroxy, un radical $-C(R^4R^5)-(CHR^6)_n-W$, $-(CH_2)_2-OCOR^{10}$ ou un radical

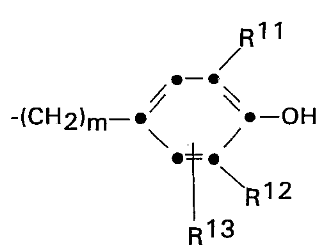

radicaux dans laquelle $R^4$, $R^5$ et $R^6$ représentent chacun, indépendamment les uns des autres, l'hydrogène, un alkyle en $C_1$-$C_6$, un phényle ou un cyclohexyle, W représente un radical -$COR^7$, -$COOR^8$, -$CON(R^9R^{15})$ ou -CN, $R^7$ représente l'hydrogène, un alkyle en $C_1$-$C_{20}$, un cycloalkyle en $C_5$-$C_{12}$, un phényle, un naphthyle-1, un naphthyle-2, un aralkyle en $C_7$-$C_{14}$, ou un alkylaryle en $C_7$-$C_{14}$, $R^8$ représente un alkyle en $C_1$-$C_{20}$, un cycloalkyle en $C_5$-$C_{12}$, un phényle, un naphthyle-1, un naphthyle-2, un aralkyle en $C_7$-$C_{14}$, un alkylaryle en $C_7$-$C_{14}$, un alkyle en $C_2$-$C_{20}$ porteur d'un hydroxy ou d'un cyano, un alcényle en $C_2$-$C_{12}$, un alcynyle en $C_2$-$C_{12}$, un phényle porteur d'un ou de deux substituants pris dans l'ensemble constitué par -$NO_2$,-Cl, -Br, -$OCH_3$ et -$COOR^{14}$, un radical de formule

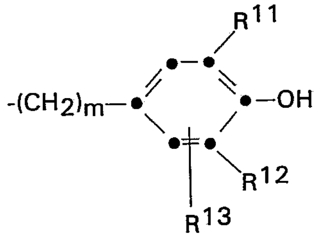

ou un alkyle en $C_3$-$C_{20}$ qui porte éventuellement un radical hydroxy qui est interrompu par 1 à 5 radicaux pris dans l'ensemble constitué par -O-, -S-, -$N(CH_3)$-, -$N(C_2H_5)$- et -$SO_2$- et dans lequel, lorsqu'il y a plusieurs hétéroatomes, ceux-ci doivent être séparés par au moins 1 radical méthylène, $R^9$ a l'une des significations qui ont été données pour $R^8$ et peut en outre représenter l'hydrogène, $R^{15}$a l'une des significations qui ont été données pour $R^9$, ou $R^9$ et $R^{15}$ forment ensemble, et avec l'atome d'azote qui les porte, un hétérocycle à 5, à 6 ou à 7 maillons qui peut éventuellement contenir un hétéroatome supplémentaire, $R^{10}$ représente un alkyle en $C_1$-$C_{20}$, un cycloalkyle en $C_5$-$C_{12}$, un phényle, un naphthyle-1, un naphthyle-2, un aralkyle en $C_7$-$C_{14}$, un alkylaryle en $C_7$-$C_{14}$ ou un radical

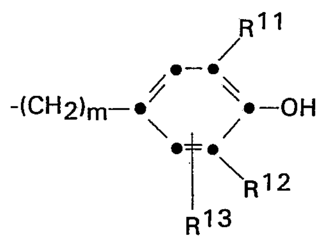

m est égal à 0, à 1 ou à 2, n est égal à 9 ou à 1, $R^{11}$, $R^{12}$ et $R^{13}$ représentent chacun, indépendamment les uns des autres, l'hydrogène, un alkyle en $C_1$-$C_{20}$, un cyclohexyle ou un phényle, et enfin $R^{14}$ représente un alkyle en $C_1$-$C_6$, un phényle, un cyclohexyle, un benzyle ou un tolyle.

2. Composés de formule I selon la revendication 1 dans lesquels $R^1$, $R^2$ et $R^3$ représentent chacun, indépendamment les uns des autres, un radical hydroxy-2 éthyle, hydroxy-2 propyle ou dihydroxy-2,3 propyle.

3. Composés de formule I selon la revendication 1 dans lesquels $R^1$, $R^2$ et $R^3$ représentent chacun, indépendamment les uns des autres, un radical $C(R^4R^5)$-$(CHR^6)_n$-W, -$(CH_2)_2$-$OCOR^{10}$ ou un radical

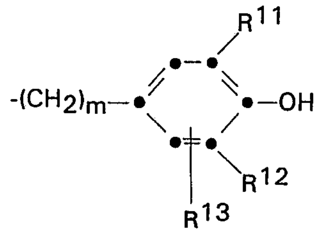

radicaux dans lesquels les symboles $R^4$, $R^5$, $R^6$, $R^{10}$, $R^{11}$, $R^{12}$, $R^{13}$, W, m et n ont les significations données à la revendication 1.

4. Composés de formule I selon la revendication 3 dans lesquels $R^4$, $R^5$ et $R^6$ représentent chacun, indépendamment les uns des autres, l'hydrogène ou un méthyle, $R^7$ représente un alkyle en $C_1$-$C_{12}$, un cycloalkyle en $C_5$-$C_9$, un phényle, un benzyle ou un tolyle, $R^8$, $R^9$ et $R^{15}$ ont chacun, indépendamment les uns des autres, la même signification que $R^7$ ou peuvent en outre représenter chacun, indépendamment les uns des autres, un radical hydroxy-2 éthyle, hydroxy-2 propyle, cyano-2 éthyle, allyle ou propyne-2 yle, un phényle porteur d'un -Cl, d'un -$COOCH_3$ ou d'un -$OCH_3$, ou un radical

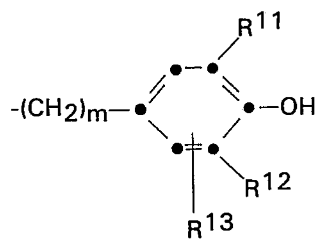

ou $R^8$, $R^9$ et $R^{15}$ représentent chacun un alkyle en $C_3$-$C_{12}$ qui est interrompu par 1 à 5 radicaux -O-, qui porte éventuellement un radical hydroxy et dans lequel, s'il contient plusieurs atomes d'oxygène, ceux-ci doivent être séparés par au moins 1 radical méthylène, ou $R^9$ et $R^{15}$ forment ensemble, et avec l'atome d'azote qui les unit, un cycle de pyrrole, de pyrrolidine, de pipéridine ou de morpholine, $R^{10}$ représente un alkyle en $C_1$-$C_{12}$, un cycloalkyle en $C_5$-$C_9$, un phényle, un aralkyle en $C_7$-$C_9$ ou un radical

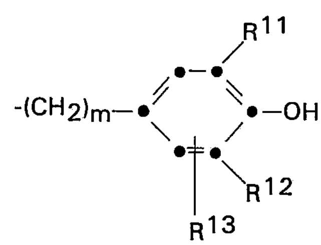

m est égal à 0, à 1 ou à 2, et enfin $R^{11}$, $R^{12}$ et $R^{13}$ représentent chacun, indépendamment les uns des autres, l'hydrogène ou un alkyle en $C_1$-$C_{12}$.

5. Composés de formule I selon la revendication 1 dans lesquels $R^4$, $R^5$ et $R^6$ représentent chacun, indépendamment les uns des autres, l'hydrogène ou un méthyle, W représente un radical $-COOR^8$ ou $-CON(R^9R^{15})$, $R^8$, $R^9$ et $R^{15}$ représentent chacun, indépendamment les uns des autres, an alkyle en $C_1$-$C_{12}$, un cyclohexyle, un phényle, un benzyle, un tolyle ou un radical de formule

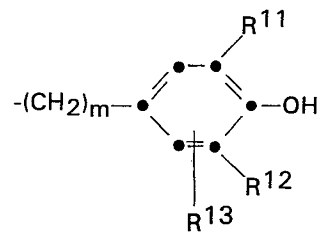

ou $R^8$, $R^9$ et $R^{15}$ représentent chacun un alkyle en $C_3$-$C_{12}$ qui est interrompu de 1 à 5 fois par un radical -O-, qui porte éventuellement un radical hydroxy et dans lequel, si il y a plusieurs atomes d'oxygène, ceux-ci doivent être séparés par au moins 1 radical méthylène, $R^{10}$ représente un alkyle en $C_1$-$C_{12}$, un cyclohexyle, un phényle, un benzyle ou un radical

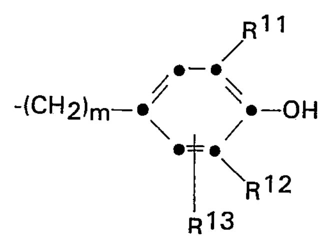

m est égal à 0, à 1 ou à 2, et $R^{11}$, $R^{12}$ et $R^{13}$ représentent chacun, indépendamment les uns des autres, l'hydrogène ou un tert.-butyle.

6. Composés de formule I selon la revendication 1 dans lesquels $R^1$, $R^2$ et $R^3$ représentent chacun un radical $-C(R^4R^5)-(CHR^6)_n-W$, $R^4$, $R^5$ et $R^6$ représentent chacun, indépendamment les uns des autres, l'hydrogène ou un méthyle, W représente un radical $-COOR^8$ ou $-CON(R^9R^{15})$, $R^8$, $R^9$ et $R^{15}$ représentent chacun, indépendamment les uns des autres, un alkyle en $C_1$-$C_{12}$, un cyclohexyle, un phényle, un benzyle, un tolyle, un radical

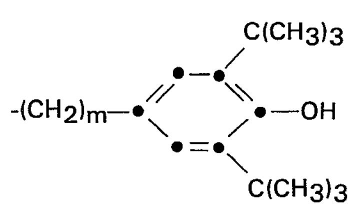

un hydroxy-2 éthyle, un hydroxy-2 propyle, un oxa-3 butyle ou un radical $-(CH_2-CH_2-O)_p-CH_2-CH_2-OH$, p est égal à 1, à 2 ou à 3, m et n ont les significations données à la revendication 1, et $R^9$ et $R^{15}$ forment ensemble, et avec l'atome d'azote qui les porte, un cycle de pipéridine ou de morpholine.

7. Composés de formule I selon la revendication 1 dans lesquels $R^1$, $R^2$ et $R^3$ représentent chacun un radical $-(CH_2)_2-OCOR^{10}$, $R^{10}$ représente un alkyle en $C_1$-$C_{12}$, un cyclohexyle, un phenyle, un benzyle ou un radical

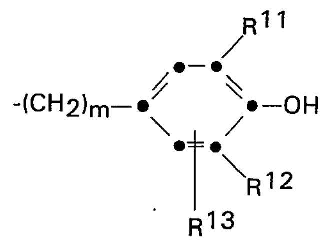

$R^{11}$, $R^{12}$ et $^{13}$ représentent chacun, indépendamment les uns des autres, l'hydrogène ou un tert.-butyle, et m est égal à 0, à 1 ou à 2.

8. Composés de formule I selon la revendication 1 dans lesquels $R^1$, $R^2$ et $R^3$ représentent chacun un radical de formule

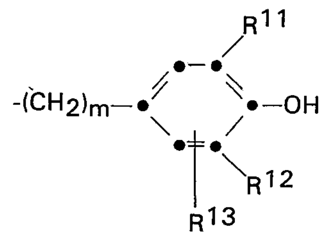

$R^{11}$, $R^{12}$ et $R^{13}$ représentent chacun, indépendamment les uns des autres, l'hydrogène ou un tert.-butyle et m est égal à 0, à 1 ou à 2.

9. Composé de formule I selon la revendication 1, en l'espèce celui dans lequel $R^1$, $R^2$ et $R^3$ représentent chacun le radical

$$-CH_2-COO-CH_2-\underset{\displaystyle CH_2CH_3}{\underset{|}{CH}}-(CH_2)_3-CH_3$$

10. Compositions qui contiennent une matière organique sensible à la dégradation par la chaleur, l'oxydation ou les rayonnements, et au moins un composé de formule I selon la revendication 1.

11. Compositions selon la revendication 9 dans lesquelles à la matière organique est un polymère ou un lubrifiant.

12. Composition selon la revendication 9 dans laquelle la matière organique est une polyoléfine, un élastomère ou un polymère du styrène.

13. Composition selon la revendication 9 dans laquelle la matière organique est une huile minérale ou un lubrifiant synthétique.

14. Application de composés de formule I selon la revendication 1 comme stabilisants pour des matières organiques à protéger contre les dégradations dues à l'action de l'oxygène, de la chaleur, de la lumière ou d'un rayonnement de haute énergie.